# EUROPÄISCHE PATENTANMELDUNG

(11) **EP 1 533 291 A1**
(43) Veröffentlichungstag der Anmeldung: **25.05.2005**
(21) Anmeldenummer: 04024607.6
(22) Anmeldetag: 15.10.2004
(51) Int. Cl.: C07C 29/70, C07C 31/30

(54) **Verfahren zur katalytischen Herstellung von Alkalialkoholaten**

(30) Priorität: 10.11.2003 DE 10352877
(71) Anmelder: BASF AKTIENGESELLSCHAFT, 67056 Ludwigshafen (DE)
(72) Erfinder: Schierle-Arndt, Kerstin, Dr., 64673 Zwingenberg (DE); Ansmann, Andreas, Dr., 69168 Wiesloch (DE); Sterzel, Hans-Josef, Dr., 67125 Danstadt-Schauernheim (DE); Schläfer, Dieter, Dr., 67071 Ludwigshafen (DE); Guth, Josef, 67251 Freinsheim (DE); Friedrich, Holger, Dr., 67240 Bobenheim-Roxheim (DE)

(57) **Zusammenfassung**

Alkalialkoholate werden hergestellt durch Umsetzung von Alkalimetallamalgam rr Alkohol in Gegenwart eines Katalysators, der poröses Eisen enthält.

## Beschreibung

Die vorliegende Erfindung betrifft ein Verfahren zur katalytischen Herstellung von Alkalialkoholaten durch Umsetzung von Alkalimetallamalgam mit einem Alkohol in Gegenwart eines Katalysators.

Alkalialkoholate (systematischer Name: "Alkalialkanolate", auch "Alkalialkoxide" ist ein gebräuchliches Synonym) sind wohlbekannte Reagenzien in der organischen Chemie. Sie werden dort, wo starke Basen als Reaktionspartner benötigt werden, und als Katalysatoren bestimmter Reaktionen eingesetzt. In größeren Mengen hergestellt und verwendet werden praktisch nur aliphatische Alkalialkoholate des Lithiums, Natriums und Kaliums mit 1 bis 4 C-Atomen im Alkylrest des Alkohols, insbesondere Lithium-, Natrium- und Kalium- -methylat, -ethylat, -n-propylat, -iso-propylat, -n-butylat und - tert.-butylat. Es sind eine Reihe von Verfahren zur Herstellung von Alkalialkoholaten bekannt. Die am weitesten verbreiteten sind die Umsetzung des Alkalimetalls mit dem Alkohol unter Wasserstoffentwicklung und die Umsetzung des Alkalimetallhydroxids mit dem Alkohol unter Abtrennung des als Nebenprodukt entstehenden Wassers. Ein Verfahren speziell zur Herstellung höherer Alkalialkoholate ist die Umsetzung eines Alkalimetallmethylats oder -ethylats mit einem höheren Alkohol unter Abtrennung von Methanol oder Ethanol. Dieses letztgenannte Verfahren und die Umsetzung von Alkalihydroxid mit Alkohol unter Abtrennung des Nebenprodukts Wasser ist der direkten Umsetzung von Alkalimetall mit Alkohol häufig aus wirtschaftlicher Sicht unterlegen, da sie vergleichsweise energieaufwendig sind.

Die direkte Umsetzung eines Alkalimetalls mit einem Alkohol ist die einfachste Herstellweise für Alkalialkoholate. Die Reaktivität der Alkalimetalle steigt in der Reihenfolge Lithium, Natrium, Kalium, Rubidium und Cäsium, und die Reaktivität der Alkohole sinkt mit dem Molekulargewicht des Alkohols und dem Verzweigungsgrad des Alkylrests. Die Umsetzung wird vorteilhafterweise mit einer Dispersion des Alkalimetalls in einem inerten Lösungsmittel oder mit Alkaliamalgam durchgeführt. Einen allgemeinen Überblick über aliphatische Alkalialkoholate, ihre Herstellung und Verwendung gibt beispielsweise Ullmann's Encyclopedia of Industrial Chemistry, Sixth Edition, Band 2, WILEY-VCH Verlag GmbH & Co. KgaA, Weinheim 2003 (ISBN 3-527-30385-5), als Punkt 4. unter dem Stichwort "Alcohols, Aliphatic". Einen Überblick über die technische Herstellung von Alkalialkoholaten aus Alkaliamalgam und dazu verwendete Reaktionsapparate gibt z. B: R. B. MacMullin in: "By-Products of Amalgam-type Chlorine Cell", Chemical Engineering Progress Sept. 1950, S. 440-455, 448.

Bei der Herstellung höherer Alkalialkoholate - im Rahmen dieser Erfindung werden darunter Alkoholate mit mindestens 3 Kohlenstoffatomen im organischen Rest verstanden - ist die von MacMullin, l.c., beschriebene Vorgehensweise zwar anwendbar, aber aufgrund niedriger Reaktionsgeschwindigkeiten häufig wirtschaftlich nicht befriedigend. Es wurden daher Verfahren entwickelt, die durch Einsatz eines Katalysators eine höhere Raumzeitausbeute ermöglichen.

Derartige katalytische Verfahren sind altbekannt. So lehrt US 2,069,403 ein Verfahren zur Herstellung von Alkalimetallalkoholaten durch Umsetzung von Alkalimetallamalgam mit Alkoholen mit bis zu 4 Kohlenstoffatomen, bei dem die Umsetzung in Gegenwart eines Katalyators aus Graphit oder Eisen-Chrom-Legierungen, die wahlweise auch weitere Legierungsbestandteile wie Nickel, Molybdän, Wolfram, Mangan enthalten können, durchgeführt wird. Eine spezielle Ausführungsform dieses Verfahrens sowie ein typischer Zersetzer (Reaktoren zur Umsetzung von Amalgam mit Alkohol oder Wasser werden üblicherweise "Zersetzer" genannt) werden in US 2,336,045 gelehrt. Dabei werden das Amalgam und der Alkohol im Gegenstrom durch den Zersetzer geführt, der den Katalysator in Form einer Packung enthält. In diesen Schriften wird der Katalysator als "Elektrode" bezeichnet.

Im Verfahren von US 2,761,880 (oder der äquivalenten DE 928 467) wird das Alkaliamalgam in Form einer Dispersion im Gegenstrom zum Alkohol eingesetzt, wobei zusätzlich Elektrodengraphit, Aktivkohle und/oder Eisenspäne als Katalysator verwendet werden. Ein bevorzugter Katalysator ist eine Mischung von Aktivkohle mit 10 - 20 % Eisenspänen. DE 973 323 offenbart einen Katalysator, der 0,1 -10 Gew.-% eines Metalls der Eisengruppe, insbesondere Eisen oder Nickel, auf einem Graphitträger enthält. EP 177 768 Al lehrt einen Katalysator zur Alkalimetallalkoholatherstellung, der aus Schwermetalloxid oder -oxiden, insbesondere einer Mischung aus Nickel- und Molybdänoxid, die auf die Oberfläche eines stückigen Anthrazitträgers aufgebracht sind, besteht. US 5,262,133 lehrt die Verwendung von Wolframcarbid, Eisen auf Kohlenstoffträger, Iridium, Ruthenium oder deren Mischungen als Katalysator.
EP 810 193 A2 offenbart Katalysatoren aus Carbiden und Nitriden von Chrom, Molybdän oder Wolfram, sowie aus Titancarbid. Im Verfahren von DE 198 02 013 Al werden Katalysatoren aus Übergangsmetallcarbiden, -nitriden oder -carbonitriden, insbesondere von Molybdän- oder Wolframcarbid, in Pulverform verwendet, und im Verfahren von EP 1 018 499 A2 wird dieser Pulverkatalysator durch Einwirkung von Ultraschall suspendiert. EP 1 195 369 Al lehrt die Verwendung eines Katalysators zur Alkaliamalgamzersetzung, der Eisen mit einem Kohlenstoffgehalt von mindestens 0,3 Gew.% enthält.

Es besteht weiterhin Bedarf an verbesserten Verfahren, insbesondere Verfahren mit verbesserter Raumzeitausbeute, wobei die verwendeten Katalysatoren möglichst preiswert sein sollen. Es bestand daher die Aufgabe, ein einfaches, wirtschaftlich befriedigendes Verfahren zu finden, das eine möglichst hohe Raumzeitausbeute ermöglicht und mit einem preiswerten Katalysator auskommt.

Demgemäß wurde ein Verfahren zur Herstellung von Alkalialkoholaten durch Umsetzung von Alkalimetallamalgam mit Alkohol in Gegenwart eines Katalysators gefunden, das dadurch gekennzeichnet ist, dass man einen Katalysator verwendet, der poröses-Eisen enthält.

Der im erfindungsgemäßen Verfahren verwendete Katalysator ist preiswert, zeigt hohe Standzeiten und ermöglicht hohe Raumzeitausbeuten. Es ist ein besonderer Vorzug des erfindungsgemäßen Verfahrens, dass es problemlos in bestehenden Zersetzern durchgeführt werden kann, so dass keine Umrüstung bestehender Anlagen erforderlich ist.

Als Alkalimetall wird im erfindungsgemäßen Verfahren Lithium, Natrium, Kalium, Rubidium oder Cäsium verwendet. Vorzugsweise werden Natrium oder Kalium verwendet. Das Alkalimetallamalgam, das das verwendete Alkalimetall enthält, kann mit jedem bekannten Verfahren zur Herstellung von Alkaliamalgam erzeugt werden, beispielsweise durch Vermischen von Alkalimetall und Quecksilber, wird aber zumeist in bekannter Weise durch Elektrolyse einer Lösung eines entsprechenden Salzes, beispielsweise eines Halogenids, in der Regel das Alkalichlorid, in einer Elektrolysezelle hergestellt. Besonders dazu geeignet ist der Typ von Elektrolysezellen, in dem das bekannte A-malgamverfahren zur Herstellung von Chlor und Natronlauge üblicherweise durchgeführt wird. Das Amalgam enthält in der Regel mindestens 0,05 Gew.-% Alkalimetall, vorzugsweise mindestens 0,1 Gew.-% und in besonders bevorzugter Weise mindestens 0,2 Gew.-%. Es enthält in der Regel höchstens 1 Gew.-% Alkalimetall, vorzugsweise höchstens 0,7 Gew.-% und in besonders bevorzugter Weise höchstens 0,5 Gew.-%.

Als Alkohol kann im Prinzip jede Verbindung mit einer Hydroxigruppe an einem kohlenstoffhaltigen Rest eingesetzt werden. Im allgemeinen werden aliphatische Alkohole eingesetzt, insbesondere solche mit einem geradkettigen oder verzweigten Kohlenstoffrest mit einem bis 8 Kohlenstoffatomen. In bevorzugter Weise werden primäre, sekundäre oder tertiäre Alkohole mit einem bis 5 Kohlenstoffatomen eingesetzt, und ein besonders bevorzugter Weise solche mit einem bis vier Kohlenstoffatomen. Beispiele für im erfindungsgemäßen Verfahren eingesetzte Alkohole sind Methanol, Ethanol, n-Propanol, iso-Propanol, n-Butanol, 2-Butanol (sec.-Butanol) 2-Methyl-1-propanol (iso-Butanol), 2-Methyl-2-propanol (tert.-Butanol), 1-, 2- oder 3-Pentanol, neo-Pentanol, tert.-Pentanol, Hexan-, Heptan- oder Octanol.

Wirtschaftlich am bedeutendsten und daher bevorzugte Produkte des erfindungsgemäßen Verfahrens sind Natrium- und Kalium- -methylat, -ethylat und -tert.-butylat. Bevorzugt ist daher die Verwendung von Methanol, Ethanol oder tert.-Butanol.

Es ist ebenso möglich, Alkohole mit mehr als einer Hydroxigruppe zu verwenden, beispielsweise Glykol.

Nachdem die Alkalialkoholate durch Substitution des Wasserstoffatoms der Hydroxigruppe des Alkohols durch das Alkalimetall gebildet werden, findet sich der Alkylrest des verwendeten Alkohols direkt im Alkalialkoholat wieder.

Das erfindungsgemäße Verfahren ist insbesondere geeignet zur Herstellung von Natriummethanolat, Kaliummethanolat, Natriumethanolat, Kaliumethanolat, Natrium-tert.butylat und Kalium-tert.-butylat.

Der Katalysator ist porös. Porosität ist die Eigenschaft eines Stoffs, Poren zu haben, sie kann daher naturgemäß nur Feststoffen zukommen. Ein Maß für die Porosität ist der Porositätsgrad, das Verhältnis aus dem Volumen der Poren in einem Formkörper zum Volumen des Formkörpers. Meist wird das Volumen der Poren jedoch als Volumen der Poren je Gramm des Stoffs angegeben, etwa in der Einheit Milliliter pro Gramm. Es kann einfach durch Aufnahme einer Fluids in den porösen Stoff bis zur Sättigung und Bestimmung der aufgenommenen Menge gemessen werden. Diese Methoden sind Routine (etwa Quecksilberporosimetrie oder Messung von Adsorptionsisothermen, üblicherweise nach dem sogenannten BET-Verfahren (für beide Verfahren sind automatisierte Analysengeräte im Handel) oder in der einfachsten Form die sogenannte "Wasseraufnahme", die Differenzwägung von trockenem und von bis zur Sättigung mit Wasser getränktem Stoff). Eine andere Möglichkeit, Porosität auszudrücken, ist durch die Oberfläche des Stoffs. Die gesamte Oberfläche eines porösen Stoffs wird im wesentlichen von Oberfläche seiner Poren gebildet (die "innere Oberfläche") und ist daher stets größer als die geometrische Oberfläche des betreffenden Formkörpers. Eine hohe Gesamtoberfläche geht daher stets mit hoher Porosität einher, sofern es sich beim betreffenden Stoff nicht um eine Menge außerordentlich feiner Partikel mit einer entsprechend hohen geometrischen Oberfläche handelt. Gemessen wird die Gesamtoberfläche eines porösen Stoffs üblicherweise durch Messung von Adsorptionsisothermen, meist nach dem sogenannten BET-Verfahren. Oft nennt man sie daher auch "BET-Oberfläche", damit ist - ohne sonstigen Hinweis - stets die spezifische Oberfläche in der Einheit Quadratmeter Oberfläche je Gramm des Stoffs gemeint. Die geometrische Oberfläche einer Eisenkugel von z.B. 2 mm Radius, die 2,6 Gramm wiegt (Dichte von Eisen = 7,873 g/cm³) beträgt beispielsweise 0,5 cm², entsprechend einer spezifischen Oberfläche von 19 · 10⁻⁶ m²/g. Für unregelmäßig geformte Formkörper oder solche mit rauher Oberfläche kann diese geometrische Oberfläche höher sein und wenige Zehntel m²/g, z. B. 0,3 m²/g erreichen. Außerordentlich feine Pulver können auch eine deutlich höhere BET-Oberfläche aufweisen, die im wesentlichen durch die äußeren Oberflächen der einzelnen Partikel gebildet wird.

Im erfindungsgemäßen Verfahren wird poröses Eisen mit einer BET-Oberfläche von im Allgemeinen mindestens 1 m²/g, vorzugsweise mindestens 2 m²/g und in besonders bevorzugter Weise mindestens 3 m²/g sowie im Allgemeinen von höchstens 100 m²/g, vorzugsweise höchstens 50 m²/g und in besonders bevorzugter Weise höchstens 20 m²/g verwendet. Ein gut geeigneter Katalysator hat beispielsweise eine BET-Oberfläche von mindestens 4 oder mindestens 6 m²/g, sowie beispielsweise höchstens 10 m²/g oder höchstens 8 m²/g.

In einer bevorzugten Ausführungsform enthält der Katalysator poröses alpha-Eisen (α-Eisen), das Eisen im Katalysator liegt in dieser bevorzugten Ausführungsform also zumindest teilweise in Form von alpha-Eisen vor. Alpha-Eisen ist eine der enantiotropen Modifikationen des Elements Eisen, nämlich die mit einer kubisch-raumzentrierten Kristallstruktur und - unterhalb seiner Curie-Temperatur von 768 °C - ferromagnetischen Eigenschaften. Alpha-Eisen ist also nicht wie Eisen mit wesentlichem Kohlenstoffgehalt (> 0.1 Gew.-% C) permanentmagnetisch.

Neben Eisen kann der Katalysator noch weitere Elemente enthalten. Dies sind einmal unvermeidbare Verunreinigungen, die aus dem Rohmaterial und etwaigen Zuschlagstoffen herrühren und beim Herstellprozess nicht oder nur unter unverhältnismäßigem Aufwand entfernt werden können, die jedoch nicht absichtlich zugesetzt werden. Andererseits können dem Katalysator auch absichtlich Zusätze zugegeben werden (sogenannte "Promotoren"), die Einfluss auf seine katalytischen Eigenschaften zeigen.

Der im erfindungsgemäßen Verfahren verwendete Katalysator enthält vorzugsweise einen oder mehrere Promotoren aus den beiden folgenden Gruppen von Promotoren:
a) mindestens einen aus der von Aluminium, Silizium, Zirkonium, Titan und Mangan und den Verbindungen dieser Elemente gebildeten Gruppe gewählten Promotor,
   und/oder
b) mindestens einen aus den Alkalimetallen (Li, Na, K, Rb, Cs) und den Erdalkalimetallen (Mg, Ca, Sr, Ba) gewählten Promotor.

Diese Promotoren liegen im Katalysator üblicherweise als Oxide oder Hydroxide vor.

Die Promotoren der Gruppe a) werden, sofern sie eingesetzt werden, jeweils in einer Menge eingesetzt, die im Allgemeinen einem Gehalt von mindestens 0,001 Gew.-%, des betreffenden Elements (d.h., Al, Si, Zr, Ti oder Mn) bezogen auf die Gesamtmasse des Katalysators, vorzugsweise mindestens 0,005 Gew.-% und in besonders bevorzugter Weise mindestens 0,01 Gew.-% sowie im Allgemeinen höchstens 3 Gew.-%, in bevorzugter Weise höchstens 1 Gew.-% und in besonders bevorzugter Weise höchstens 0,3 Gew.-% entspricht.

Die Promotoren der Gruppe b) werden, sofern sie eingesetzt werden, jeweils in einer Menge eingesetzt, die im Allgemeinen einem Gehalt von mindestens 0,0001 Gew.-%, des betreffenden Elements (d.h., Alkali- oder Erdalkalimetall) bezogen auf die Gesamtmasse des Katalysators, vorzugsweise mindestens 0,0005 Gew.-% und in besonders bevorzugter Weise mindestens 0,001 Gew.-% sowie im Allgemeinen höchstens 3 Gew.-%, in bevorzugter Weise höchstens 0,5 Gew.-% und in besonders bevorzugter Weise höchstens 0,25 Gew.-% entspricht.

Das alpha-Eisen sollte möglichst wenig homogen dispers in das metallische Gefüge eingebauten Kohlenstoff (auch "gelöster" Kohlenstoff genannt) enthalten. In bevorzugter Weise beträgt sein Gehalt an derartigem Kohlenstoff höchstens 0,1 Gew.-%, bezogen auf die Menge des alpha-Eisens, und in besonders bevorzugter Form höchstens 0,05 Gew.%. Es ist jedoch möglich, dass im Katalysator aus dem metallischen Gefüge ausgeschiedener Kohlenstoff, typischerweise in Form von größeren, losen oder inhomogen verteilten Graphitpartikeln vorliegt. Diese beeinflussen das Verfahren nicht wesentlich.

Prinzipiell kann jeder auf Eisen basierende Katalysator zur Synthese von Ammoniak aus Stickstoff und Wasserstoff im erfindungsgemäßen Verfahren verwendet werden. Ammoniakkatalysatoren auf Eisenbasis, ihre Herstellung und Anwendung sind wohlbekannt. Eine Übersicht über Ammoniakkatalysatoren, ihre Herstellung und Anwendung gibt beispielsweise Ullmann's Encyclopedia of Industrial Chemistry, Sixth Edition, Band 2, WILEY-VCH Verlag GmbH & Co. KgaA, Weinheim 2003 (ISBN 3-527-30385-5), als Punkt 4. unter dem Stichwort "Ammonia". Für das erfindungsgemäße Verfahren geeignete Katalysatoren sind auch handelsüblich und als Ammoniakkatalysatoren - entweder als Eisenoxid enthaltender Katalysatorvorläufer oder bereits als vorreduzierter und passivierter Katalysator - etwa von der Firma Haldor Topseøe A/S, Lyngby, Dänemark, unter den Bezeichnungen KM1 oder - in reduzierter und passivierter Form - KM1 R erhältlich, von der Firma Synetix, Billingham, England (ein Tochteruntemehmen der Johnson Matthey plc), unter den Bezeichnungen S6-10, 35-4 oder 74-1 oder, jeweils in vorreduzierter und passivierter Form, S6-10R, 35-8 oder 74-1 R, oder von Süd-Chemie AG, München, Deutschland, unter den Bezeichnungen AS-4 oder - vorreduziert und passiviert - AS-4F. Die Typen S6-10 und S6-10R der Synetix wurden bis 1996 von BASF Aktiengesellschaft vertrieben, so dass sie in manchen Literaturübersichten noch als Katalysatoren der BASF bezeichnet werden.

Der Katalysator wird mit den für die Herstellung von Ammoniakkatalysatoren bekannten Methoden wie diese aus Eisen enthaltenden Rohstoffen hergestellt. Geeignete Rohstoffe sind Eisenoxide oder Eisenerze, insbesondere solche, in denen das Eisen zu 90 bis 100 Gew.-% in Form von Eisenoxid, Eisenhydroxid, Eisenoxihydroxid oder deren Gemischen vorliegt. Bevorzugt ist die Verwendung von synthetisch hergestellten oder natürlich vorkommendem Brauneisenstein, Roteisenstein (Hämatit), Wüstit oder Magneteisenstein (Magnetit) oder Gemischen davon. Manche der natürlich vorkommenden Rohstoffe enthalten die für den Katalysator geeigneten Promotoren in den richtigen Mengen, ansonsten werden ihnen geeignete Vorläuferverbindungen der Promotoren wie etwa ihre Oxide oder Hydroxide zugesetzt.

Werden die Eisenoxid-Rohstoffe synthetisch hergestellt, so kann man von sehr reinem metallischem Eisen oder von sehr reinen Eisen (II)- und/oder Eisen (III)-Verbindungen ausgehen, denen die ausgewählten Promotoren in der entsprechenden Menge zugesetzt werden. Diese Verfahren sind bekannt. Typischerweise werden dazu mindestens ein lösliches Eisensalz sowie lösliche Salze der gewünschten Promotoren in einem Lösungsmittel, in bequemer Weise Wasser, gelöst und ein Eisen enthaltener Rohstoff zur Herstellung des Katalysators durch Fällung (etwa durch Erhöhung der Temperatur oder des pH-Werts der Lösung oder Zugabe von Reduktionsmitteln) abgeschieden. Geeignete lösliche Salze von Eisen und Promotoren sind beispielsweise ihre Nitrate, Chloride, Acetate, Formiate und Sulfate, vorzugsweise die Nitrate.

Üblicherweise trocknet man die so erhaltenen Katalysatorvorläufer bei Temperaturen im Bereich von 80 bis 150°C, vorzugsweise im Bereich von 80 bis 120°C, und kalziniert anschließend bei Temperaturen im Bereich von 150 bis 500°C, vorzugsweise im Bereich von 200 bis 450°Cin einem Gasstrom aus Luft oder Stickstoff vor.

Vor oder nach der Trocknung oder Kalzinierung nimmt man die Formgebung der Katalysatorvorläufer vor, üblicherweise durch Knetung und Extrusion oder Tablettierung. Eine weitere Möglichkeit, derartige Katalysatoren herzustellen, besteht im Aufschmelzen der Eisenkomponenten zusammen mit den Promotoren aus den Gruppen a) und b) und der anschließenden Zerkleinerung des Schmelzkuchens zu Splitt. Das Aufschmelzen kann nach allgemein bekannten Methoden, beispielsweise durch Widerstandsheizung oder induktiv erfolgen. Nach dem Erkalten des Schmelzkuchens wird dieser mittels geeigneter Apparate wie beispielsweise Backenbrechem bis zu einer gewünschten Feinheit zerkleinert, wobei Über- und Unterkom abgesiebt wird. Der Katalysator wird im erfindungsgemäßen Verfahren als Pulver oder vorzugsweise in stückiger Form eingesetzt. Partikelgrößen mit Abmessungen in den drei Raumrichtungen von mindestens 0,5 mm, vorzugsweise 1 mm, und höchstens 15 mm, vorzugsweise höchstens 10 und in besonders bevorzugter Weise höchstens 7 mm, sind in aller Regel gut zur Verwendung in gängigen Amalgamzersetzem geeignet. Um die Bildung regelmäßiger räumlicher Strukturen zu vermeiden, werden vorzugsweise unregelmäßig geformte Partikel eingesetzt, am bequemsten Splitt.

Der Katalysatorvorläufer, in dem das Eisen im wesentlichen als Eisenoxid, Eisenhydroxid und/oder Eisenoxihydroxid vorliegt, wird durch Reduktion in einen alpha-Eisen enthaltenden porösen Katalysator umgewandelt. Diese Reduktionsverfahren sind ebenfalls von Ammoniakkatalysatoren bekannt. Die Reduktion des Katalysatorvorläufers zum metallischen Eisen wird meist als "Aktivierung" bezeichnet. Man setzt dazu typischerweise den Katalysatorvorläufer einer reduzierenden Atmosphäre aus, beispielsweise indem man sie bei einer Temperatur im Bereich von 300 bis 500°C, vorzugsweise von 340 bis 450°C mit Wasserstoff oder mit einem Stickstoff-Wasserstoff-Gemisch behandelt. Die Behandlungsdauer liegt üblicherweise im Bereich von 12 bis 120 Stunden, bevorzugterweise im Bereich von 24 bis 96 Stunden. Die Katalysatorbelastung mit Gas bei der Aktivierung beträgt typischerweise 1 000 bis 10 000 Nl/kg Katalysator*h. Die Aktivierung des Katalysators kann direkt im Amalgamzersetzer durchgeführt werden. Es ist jedoch meist bequemer, einen vorreduzierten und passivierten Katalysator in den Zersetzer zu füllen und diesen vorreduzierten und passivierten Katalysator im Zersetzer lediglich erneut zu aktivieren. Auch dies ist von Ammoniakkatalysatoren wohlbekannt. In diesem Fall wird im Anschluss an die Aktivierung des Katalysators in einem dafür geeigneten Reaktor ein Luft-/Stickstoffgemisch über den Katalysator geleitet, üblicherweise bei Temperaturen von höchstens 100°C, bevorzugterweise höchstens 80°C und der Katalysator so durch Ausbildung einer dünnen Oxidschicht an seiner Oberfläche passiviert. In diesem Zustand ist er bequem handhabbar (unpassiviertes alpha-Eisen kann pyrophor sein) und kann bequem in den Zersetzer eingebracht werden. Die erneute Aktivierung des passivierten Katalysators im Zersetzer erfolgt wie die Aktivierung selbst, benötigt aber nur geringe Wasserstoffmengen und eine kürzere Zeitdauer als die ursprüngliche Aktivierung des oxidischen Katalysators. Typischerweise wird der passivierte Katalysator bei Temperaturen im Bereich von 300 bis 500°C, vorzugsweise im Bereich von 320 bis 400°C mit Wasserstoff oder einem Stickstoff-Wasserstoff-Gemisch erneut aktiviert. Die Behandlung erfolgt üblicherweise über eine Dauer im Bereich von 6 bis 72 Stunden, bevorzugterweise im Bereich von 12 bis 48 h. Die Katalysatorbelastung mit Gas bei der Reaktivierung beträgt im allgemeinen 1 bis 200 Nl/kg Katalysator*h, vorzugsweise 25 bis 75 Nl/kg Katalysator*h.

Der Katalysator wird in einem üblichen Zersetzer eingesetzt.

Zur Durchführung des erfindungsgemäßen Verfahrens werden im mit Katalysator gefüllten Zersetzer das Alkalimetallamalgam und der Alkohol umgesetzt. Üblicherweise wird der Alkohol im Überschuss eingesetzt, so dass eine Lösung des Alkalialkoholats im entsprechenden Alkohol hergestellt wird, da die Abtrennung der in Reinsubstanz bei Umgebungstemperatur festen Alkalialkoholate vom Quecksilber oder - bei unvollständiger Umsetzung des Alkalimetalls - alkaliärmeren Amalgam ansonsten nur umständlich durchzuführen wäre. Das Verfahren kann diskontinuierlich oder kontinuierlich durchgeführt werden, die kontinuierliche Verfahrensweise ist in aller Regel wirtschaftlicher. Typischerweise werden dazu der Alkohol und das Amalgam kontinuierlich im Gegenstrom durch den mit Katalysator gefüllten Zersetzer geleitet.

Das den Zersetzer verlassende Quecksilber wird wieder in die Alkalimetallamalgamherstellung zurückgeführt. Bei unvollständiger Umsetzung des Alkalimetalls kann das alkaliärmere Amalgam ebenso wieder in die Alkaliamalgamherstellung zurückgeführt werden, es kann wahlweise jedoch auch in den Reaktor zurückgeführt werden, bis der gewünschte Alkalimetallumsatz erreicht ist. Es ist ebenso möglich, den Zersetzer verlassendes alkaliärmeres Amalgam mit einem anderen Alkohol zu einem Alkoholat und Quecksilber oder mit Wasser zu Alkalihydroxid und Quecksilber umzusetzen.

Die den Zersetzer verlassende Alkalialkoholatlösung wird entweder direkt verwendet, oder das enthaltene Alkalialkoholat wird mittels üblicher Methoden isoliert (beispielsweise durch Eindampfen der Lösung und wahlweise anschließende Reinigung, beispielsweise durch Umkristallisation), oder sie wird in den Zersetzer zurückgeführt, bis die gewünschte Konzentration an Alkalialkoholat erreicht wird. Vorzugsweise wird der Alkohol-Mengenstrom durch den Reaktor so eingestellt, dass eine Alkoholatlösung der gewünschten Konzentration ohne Rückführung aus dem Zersetzer entnommen wird.

Die Reaktionstemperatur beträgt im allgemeinen mindestens 0°C, vorzugsweise mindestens 10°C und in besonders bevorzugter Weise mindestens 20°C. Da sich das Alkalialkoholat bei zu hohen Temperaturen zersetzen kann, kann die Reaktionstemperatur nicht beliebig hoch gewählt werden, ihre Obergrenze ist die Temperatur, bei der durch Zersetzung in wirtschaftlich nicht mehr tolerierbarer Menge Produktverlust eintritt. Häufig und in bevorzugter Weise wird die Reaktion bei der Siedetemperatur des verwendeten Alkohols durchgeführt.

Unter Umständen, vor allem bei der Umsetzung reaktiverer Alkohole (deren Reaktivität sinkt bekanntlich mit dem Molekulargewicht des Alkohols und mit dem Verzweigungsgrad des Substituenten) ist die Abführung der Reaktionswärme erforderlich, was durch übliche Kühlvorrichtungen erfolgt. Die Reaktionswärme wird beispielsweise durch Kühlschlangen im oder am Reaktor, Führung der den Reaktor verlassenden Stoffströme durch Wärmetauscher, oder durch Siede- oder Rückflußkühlung durch absiedenden Alkohol abgeführt. Der als Nebenprodukt entstehende Wasserstoff wird gasförmig aus dem Zersetzer abgeleitet, er wird entsorgt, beispielsweise durch Verbrennung, oder vorzugsweise verwertet, beispielsweise als Hydrierwasserstoff für Hydrierreaktionen.

Die Katalysatormenge (d.h., auch die Zersetzergröße), die Verweilzeit der Reaktanden im Reaktor und die Reaktionstemperatur werden so gewählt, dass eine etwaige Zersetzung von Einsatzstoffen oder Produkten vermieden wird oder in wirtschaftlich tolerierbarem Rahmen bleibt.

### Beispiele

### Beispiel 1: Herstellung eines erfindungsgemäßen Katalysators

Magnetiterz wurde bei einer Temperatur von mindestens 1550°C an Luft aufgeschmolzen. Der abgekühlte Schmelzblock wurde in einem Backenbrecher zerkleinert. Eine Siebfraktion mit einer Partikelgröße von 1,5-3 mm wurde ausgesiebt und in einem aus Wasserstoff und Stickstoff bestehenden Gasstrom binnen 72 h bei 450°C reduziert. Nach Abkühlen unter Stickstoff wurde der Katalysatorvorläufer mit einem Gemisch aus 1 Vol.% Luft in Stickstoff binnen 24 h passiviert, wobei die durch die Katalysatorschüttung geleitete Gasmenge so geregelt wurde, dass an keiner Stelle der Schüttung die Temperatur 45°C überstieg. Vor dem Einsatz zur Amalgamzersetzung wurde der Katalysator mit Wasserstoff bei 340°C über 46 h reduziert. Der so erhaltene reduzierte Katalysator hatte die Zusammensetzung: 0,08 Gew.-% Al, 0,17 Gew.-% Mn, 0,12 Gew.-% Si, 0,01 Gew.-% Ti, 0,03 Gew.-% Ca, 0,05 Gew.-% Mg, 0,04% C (nicht als Graphit vorliegend), Rest Fe, und seine BET-Oberfläche betrug 6 m²/g.

### Beispiel 2: Amalgamzersetzung im Laborversuch

In einem Glasreaktor (senkrecht stehendes Rohr) wurde eine Schüttung von 500 ml des Katalysators aus Beispiel 1 vorgelegt. Von oben wurde durch diese Schüttung Kaliumamalgam (0,411 Gew.-% K in Hg) mit Durchflussgeschwindigkeit von 6 l/h geleitet. Gleichzeitig wurden 400 g t.-Butanol mit einer Durchflussgeschwindigkeit von 10 l/h im Kreis durch die Kolonne gepumpt. Der Reaktionsfortschritt wird durch Säure-Base-Titration von Proben der Alkoholphase bestimmt. Die Reaktion war nach 2,2 Stunden beendet. Die Kalium-tert.-butanolat-Lösung wurde abgelassen und die Menge des erzeugten Kalium-tert.-butanolats durch Säure-Base-Titration zu 0,76 Mol bestimmt. Die Menge des als Nebenprodukt entstehenden Kaliumhydroxids wurde mittels Karl-Fischer-Titration bestimmt. Es ergibt sich eine Raumzeitausbeute von Kalium-tert.butanolat von 77 g/h*I.

### Beispiel 3: Amalgamzersetzung im Laborversuch

Beispiel 2 wurde mit einem Kaliumamalgam mit 0,340 Gew.-% K in Hg wiederholt. Binnen einer Reaktionsdauer von 1,7 Stunden entstanden 0,51 Mol Kalium-tert.-butanolat, entsprechend einer Raumzeitausbeute von 67 g/h*I.

### Beispiel 4: Amalgamzersetzung im Laborversuch (Vergleichsbeispiel)

Es wurde wie in Beispiel 2 verfahren, jedoch mit 375 ml unporösen Eisens als Katalysator (> 98 % Fe, 1,4 % C) in Form von Splitt mit einer Partikelgröße von < 2 mm und einer BET-Oberfläche von 0,3 m²/g und mit einem Kaliumamalgam mit 0,393 Gew.-% K in Hg. Binnen einer Reaktionsdauer von 15 Stunden entstanden 0,62 Mol, entsprechend einer Raumzeitausbeute von 12 g/h*I.

### Beispiel 5: Amalgamzersetzung im Laborversuch (Vergleichsbeispiel)

Es wurde wie in Beispiel 2 verfahren, jedoch mit 500 ml unporösen Eisens als Katalysator (> 99 Gew.-% Fe, < 0,2 Gew.-% C) in Form von Splitt mit einer Partikelgröße von 1-3 mm und einer BET-Oberfläche von 0,2 m²/g und mit einem Kaliumamalgam mit 0,393 Gew.-% K in Hg. Binnen einer Reaktionsdauer von 15 Stunden entstanden 0,15 Mol, entsprechend einer Raumzeitausbeute von 2 g/h*I.

Die Beispiele zeigen, dass mit dem erfindungsgemäßen Verfahren eine erhebliche Steigerung der Raumzeitausbeute erreicht wird, so dass entweder (was zumindest bei Neuinvestitionen vorteilhaft sein kann) die Zersetzer bei konstanter Produktionsleistung erheblich kleiner oder in geringerer Stückzahl ausgeführt werden können, wodurch auch der Katalysatorbedarf sinkt, oder alternativ eine erheblich höhere Produktionsleistung erreicht wird. Dies steigert die Wirtschaftlichkeit des Verfahrens ganz erheblich.

## Patentansprüche

1. Verfahren zur Herstellung von Alkalialkoholaten durch Umsetzung von Alkalimetallamalgam mit Alkohol in Gegenwart eines Katalysators, **dadurch gekennzeichnet, dass** man einen Katalysator verwendet, der poröses Eisen enthält.

2. Verfahren nach Anspruch 1, **dadurch gekennzeichnet, dass** der Katalysator eine BET-Oberfläche von mindestens 1 m²/g aufweist.

3. Verfahren nach Anspruch 1, **dadurch gekennzeichnet, dass** der Katalysator alpha-Eisen enthält.

4. Verfahren nach Anspruch 2, **dadurch gekennzeichnet, dass** der Katalysator alpha-Eisen enthält.

5. Verfahren nach Anspruch 4, **dadurch gekennzeichnet, dass** der Katalysator ein handelsüblicher Katalysator zur Ammoniaksynthese in seiner reduzierten Form ist.

6. Verfahren nach den Ansprüchen 1 bis 5, **dadurch gekennzeichnet, dass** man einen aliphatischen primären, sekundären oder tertiären Alkohol mit einem bis 8 Kohlenstoffatomen im Alkylrest verwendet.

7. Verfahren nach Anspruch 6, **dadurch gekennzeichnet, dass** man Methanol, Ethanol oder tert.-Butanol verwendet.

8. Verfahren nach Anspruch 6, **dadurch gekennzeichnet, dass** man als Alkalimetall Natrium oder Kalium einsetzt.

9. Verfahren nach Anspruch 7, **dadurch gekennzeichnet, dass** man als Alkalimetall Natrium oder Kalium einsetzt.
